Europäisches Patentamt

European Patent Office

Office européen des brevets

(11) Publication number: **0 020 781**
A1

## (12) EUROPEAN PATENT APPLICATION

published in accordance with Art. 158(3) EPC

(21) Application number: 79901576.3

(22) Date of filing: 21.11.79

Data of the international application taken as a basis:

(86) International application number: PCT/JP 79/00298

(87) International publication number: WO 80/01034 (29.05.80 80/12)

(51) Int. Cl.³: **A 23 C 21/02**, A 23 C 9/12, A 23 C 17/02, C 12 P 1/00 // A23L1/236

(30) Priority: 24.11.78 JP 145686/78

(43) Date of publication of application: 07.01.81 Bulletin 81/1

(84) Designated Contracting States: DE FR GB NL

(71) Applicant: MORINAGA MILK INDUSTRY CO., LTD., 33-1, Shiba 5-chome, Minato-ku, Tokyo 108 (JP)

(72) Inventor: OGASA, Katsuhiro, 27-5, Nagatasannodai Minami-ku, Yokohama-shi Kanagawa-ken 232 (JP)
Inventor: SHIMAMURA, Seiichi, 1558, Shinohara-cho Kohoku-ku, Yokohama-shi Kanagawa-ken 222 (JP)
Inventor: KUDO, Tsutomu, 4-74, Wakakusadai Midori-ku, Yokohama-shi Kanagawa-ken 227 (JP)
Inventor: HIRAMATSU, Akinori, 8-4-301, Kashima, Hachioji-shi Tokyo 192-03 (JP)
Inventor: MIYAKAWA, Hiroshi, 4-7-21, Ofuna, Kamakura-shi Kanagawa-ken 247 (JP)

(74) Representative: Baillie, Iain Cameron et al, c/o Ladas & Parry Blumenstrasse 48, D-8000 München 2 (DE)

(54) PROCESS FOR PRODUCING A SOLUTION OF LACTOSE DECOMPOSITION PRODUCT.

(57) A lactose-containing solution such as a lactose aqueous solution, wherein milk or the like is placed in contact with a $\beta$-galactosidase-containing solution, with a dialysis membrane intervening therebetween to decompose lactose in the solution. Thus, a $\beta$-galactosidase-free solution of the lactose decomposition product is obtained. This enzymatic reaction is conducted at temperatures not higher than the optimal temperature of used enzyme, preferably 0–10 °C, with the amount of enzyme being 30 to 250 units, preferably 62.5–250 units per 1 g of lactose in the lactose-containing solution. The area of the dialysis membrane intervening between the enzyme-containing solution and the substrate-containing solution is preferably selected so that it actually becomes largest with respect to the volume of the enzyme-containing solution used.

TITLE OF THE INVENTION

Method for Preparation of Liquid **TITLE MODIFIED**

Containing Lactose-Hydrolyzate


FIELD OF THE ART

The present invention relates to a method for
preparation of liquid containing lactose-hydrolyzate (hereinafter referred to as lactose-hydrolyzate liquid) by β-
galactosidase, and more particularly relates to a method
wherein a dialysis membrane is interposed between a liquid
containing lactose and a liquid containing β-galactosidase,
thereby lactose contained in the liquid is selectively
hydrolyzed and thus lactose-hydrolyzate liquid is obtained
without β-galactosidase migrating and/or remaining therein.

Lactose-hydrolyzate liquid is useful as a sweetening
agent, and milk and milk products in which lactose is hydrolyzed
are useful for both lactose-intorelant infants and adults.

In recent years, hydrolysis of lactose by
β-galactosidase has ben highlighted in the field of milk
industry, and in fact cow milk in which lactose is hydrolyzed
has come to be available in the market.  W. L. Wendorff
et al report that lactose in milk and milk products is
hydrolyzed by adding β-galactosidase of yeast thereto in
Journal of Milk and Food Technology, Vol. 34, No. 6 (1971),
pp 294 - 299.  Also, in Journal of the Society of Dairy
Technology, Vol. 27, No. 3 (July, 1974), pp 121 - 127, S. G.

- 1 -

0020781

Cotton refers to an experiment to produce a sweetening agent by hydrolysis of lactose of deproteinized whey.

Hitherto it has been known two types of method for hydrolysis of lactose contained in liquid by β-galactosidase. The first method is to add β-galactosidase directly to the liquid containing lactose (hereinafter referred to as the direct method), and another method is to treat lactose containing liquid with immobilized β-galactosidase. As for the known references relating to preparation of lactose-hydrolyzate liquid and to immobilized enzymes, following are hereby made records:

Journal of Dairy Science, Vol. 55, No. 5 (1972), pp 670 - 671;

Japanese Patent Public Disclosure ("Tokukai" in abbreviation in Japanese), No. 47(1972)-9722;

Japanese journal "Nutrition and Foods", Vol. 28, No. 1 (1975), pp 33 - 38;

Biotechnology and Bioengineering, Vol. 15 (1973), pp 395 - 402;

Journal of Food Science, Vol. 39 (1974), pp 374 - 378;

New Food Industry, Vol. 16, No. 9 (1974), pp 18 - 25;

Journal of Dairy Science, Vol. 56, No. 9 (1973), pp 1123 - 1127.

- 2 -

As for the utilization of dialysis membrane, there has been known a method, so-called "Dialysis Culture Method" in which dialysis membrane is utilized for cultivation of microorganisms.  This dialysis culture method has been utilized for preparation of microbial cells, microbial enzymes and microbial metabolites, preparation of fermented milk, beer and wine (see Bacteriological Reviews, Vol. 33, No. 1 (Mar. 1969), pp 1 - 47;

Japanese Patent Publication Gazettes No. 49(1974)-1968, No.

53(1978)-1348, No. 51(1976)-33198 and No. 51(1976)-37359).

Dialysis membrane has also been customarily utilized for dialytic refinement of enzymes.

TECHNOLOGICAL BACKGROUND

The direct method of adding $\beta$-galactosidase, previously

mentioned, has been found to be satisfactory in general for the efficiency of hydrolysis of lactose.  However, it is very difficult to selectively remove only the enzymes from the lactose-hydrolyzate liquid obtained by the direct method, and when this is tried, other substances contained in the lactose-hydrolyzate liquid tend to be removed along with the enzymes.

This means that either a foreign protein (enzymes) is added to the lactose-hydrolyzate liquid or some components in the lactose-hydrolyzate liquid are removed.  In certain kinds of milk products, however, it is prohibited (by regulations) to add any foreign substances thereto or to remove any components therefrom, and in such circumstances, this direct method cannot be utilized.  In addition, in this direct method, repeated use of enzymes is not possible, even if the enzymes maintain sufficient activity.  This necessitates the use of a larger amount of enzymes for preparation of lactose-hydrolyzate liquid and requires a higher production cost.

In order to eliminate mixture of enzymes in the lactose-hydrolyzate liquid and to enable repeated use of enzymes, the use of immobilized enzymes has been suggested. However, as mentioned in the references listed above, there are some problems in this method; complexity in production of immobilized β-galactosidase is one, and decrease of enzyme activity in the process of immobilization of enzymes is another. Furthermore, use of immobilized enzymes is not necessarily satisfactory, when a portion of immobilized enzyme is released and/or migrates from the carrier, qhich absorbs the enzyme, into the lactose-hydrolyzate liquid and thus reproduction (or reactivation) of immobilized enzymes becomes impossible which occurs depending upon the method for immobilization of enzymes (Chemical Industry, Vol. 38, No. 5 (1974), pp 357 - 363).

Immobilized enzyme of the type in which enzymes are entrapped within microcapsules has also been known (Japanese journal "Chemistry and Biology", Vol. 7, No. 3 (1969), pp 147 - 155). As to the application of such microcapsulated enzymes, however, a merely experimental application is known wherein catalase entrapped within micro-capsules made of semipermeable corrosion membrane and having a size of 5 - 15 μm in diameter is dispersed into the blood stream of acatalasaemic animals (Nature, Vol. 218 (1968), pp 243 - 255). It has not yet been known that such micro-capsulated enzymes are used in food industry, especially in milk industry. Even if micro-capsulated enzymes are used for hydrolysis of lactose contained

- 4 -

in liquid, it is impossible to selectively separate only the microcapsulated enzymes from the lactose-hydrolyzate liquid due to the same reason as was mentioned with respect to the direct method.

Furthermore, it has been known that in case the liquid containing lactose to be treated contains protein, activity of β-galactosidase is affected by the protein (Journal of Food Science, Vol. 39 (1974), pp 88 - 93).

Also when crude enzyme is used, there are occasions that the taste of the lactose-hydrolyzate liquid may be deteriorated due to decomposition of other substances such as protein which may be contained in the liquid by other enzymes such as protease which may be contained in the crude enzyme as one of impurities. Thus, in case the lactose-hydrolyzate liquid is used for food, use of highly refined enzyme is required. This also causes the production cost to be increased. There are some reports wherein use of unrefined enzymes may enhance pleasant taste to the products, but in case a specific taste which is different from that of the raw material is not desired, the use of refined enzyme is necessary.

In order to overcome the defects of the conventional methods, the inventors have conducted many researches, and found that when a dialysis membrane is interposed between liquid containing lactose and liquid containing β-galactosidase, lactose can be selectively and efficienctly hydrolyzed without affecting the remaining ingredients in the liquid containing lactose, thereby lactose-hydrolyzate liquid having good taste can be obtained at a low cost.

According to the present invention, there is no migrating nor remaining of β-galactosidase in the lactose-hydrolyzate liquid, and repeated use of β-galactosidase is made possible. moreover, even if protein which may decrease the enzyme activity is included in the liquid containing lactose, activity of β-galactosidase is not affected by the protein. Furthermore, when the lactose·containing liquid which also contains protein is treated in accordance with the present invention using crude β-galactosidase containing protease, the protein is not hydrolyzed (since the protein cannot pass through the dialysis membrane), and palatable lactose-hydrolyzate liquid can be obtained.

As for the utilization of dialysis membrane, production of microbial cells, microbial enzymes and microbial metabolites by dialysis culture has been known as previously mentioned, and dialysis membrane has been utilized as a customary means for refining enzymes. Also it has been desired to provide a method of hydrolysis of substrate by enzyme without inclusion of the enzyme in the substrate-hydrolyzate. Notwithstanding, there has not been a technical idea that dialysis membrane is interposed between liquid containing enzyme and liquid containing substrate to obtain edible liquid which contains enzymatically hydrolyzed substrate.

Accordingly, it is an object of the present invention to provide a method of preparation of lactose-hydrolyzate liquid using dialysis membrane.

Another object of the present invention is to provide a method of preparation of lactose-hydrolyzate liquid without

- 6 -

migrating and/or remaining of β-galactosidase in the lactose-hydrolyzate liquid to be obtained.

A further object of the present invention is to provide a method of preparation of lactose-hydrolyzate liquid wherein β-galactosidase may be used repeatedly.

Another object of the present invention is to provide a method of preparation of lactose-hydrolyzate liquid wherein lactose of liquid containing lactose and protein is selectively hydrolyzed by crude β-galactosidase which contains protease without hydrolyzing the protein.

A still further object of the present invention is to provide low-lactose milk or lactose-hydrolyzed milk as well as palatable low-lactose milk products or lactose-hydrolyzed milk products at a low cost wherein lactose in the liquid which contains lactose and other substances is selectively hydrolyzed by crude β-galactosidase without affecting said other substances.

DISCLOSURE OF THE INVENTION

The present invention relates to a method for preparation of lactose-hydrolyzate liquid by interposing dialysis membrane between liquid containing lactose and liquid containing β-galactosidase, selectively hydrolyzing lactose without substantially affecting other substances in the liquid containing lactose, and at the same time preventing β-galactosidase from migrating into the lactose-hydrolyzate liquid which is to be obtained.

BRIEF EXPLANATION OF THE DRAWINGS

0020781

Fig. 1 shows hydrolyzing rate of lactose by treating with various amounts of enzyme as a function of hydrolyzing period.

Fig. 2 shows hydrolyzing rate of lactose by holding at various temperatures as a function of hydrolyzing period.

THE PREFERRED EMBODIMENTS FOR PRACTICING THE INVENTION

For better understanding of the prevent invention, the materials used in this invention, i.e. β-galactosidase, dialysis membrane and liquid containing lactose as well as a general embodiment will be first explained, and then the present invention will be further described enumerating several tests which exemplify the features of the present invention and some examples thereof.

In the present invention, the disrupted cells of lactic acid bacteria as a crude enzyme, the enzyme refined therefrom or the enzyme on the market, like Maxi lact "40,000" (Trademark; by Gist-Brocades of the Netherlands), Sumi lact-L (Trademark; by Shin-Nihon Kagaku Kogyo Co., Ltd. of Japan), GODO-YNL (Trademark; Godo Shusei Co., Ltd. of Japan) may be used as a source of β-galactosidase. When the enzyme on the market is used, its activity is desired to be as high as possible. Commercially available β-galactosidase (which is usually sold in the form of powder) may be dispersed and dissolved in an aseptic atmosphere in distilled water or aqueous solution of lactose both sterilized and chilled, and this obtained liquid containing β-galactosidase is filled into a sterilized bag or tube one end of which is sealed, and preferably it is subjected to aseptic dialysis against sterilized distilled water to thereby preliminarily remove dializable inpurities which may be included therein.

The quantity of β-galactosidase to be used ranges 30 - 250 units per gram of lactose contained in the liquid to be treated, and preferably 125 - 250 units per gram of lactose. The term "unit" used herein indicates the activity of β-galactosidase measured by Lederberg's method (Journal of Bacteriology, Vol. 60 (1950), p. 381), and one unit means the amount of the enzume necessary for hydrolyzing 1 μ mole of orthonitrophenol-β-galactopyranoside per minute at $30^{o}$C. Usually, β-galactosidase powder on the market has enzyme activity in the range of 5,000 - 30,000 units per gram. Therefore, in case of enzyme having 5,000 units/gram activity, the quantity of the enzyme to be used will be 6 - 50 mg/gram of lactose contained in the liquid to be treated, and preferably be 25 - 50 mg/gram, and in case of enzyme having an activity of 30,000 units/gram, the quantity of the enzyme to be used will be 1 - 8.3 mg/gram of lactose, and preferably be 4.1 - 8.3 mg/gram.

Meanwhile, it has been known that β-galactosidase may have specificity due to its origin ("Enzymes in Food and Beverage Processing", pp 67 - 79, edited by Robert L. Oray, American Chemical Society, Washington D. C., 1977; New Food Industry, Vol. 16, No. 9 (1974), pp 18 - 25). For instance, the optimum pH of β-galactosidase from different origins extends over 4 - 7 and their optimum temperature extends over $35^{o}$C (originating from Saccharomyces lactis) - $55^{o}$C (originating from Aspergillus niger).

Since the pH of the liquid containing lactose used in the present invention usually extends over 6 - 7, it is preferable to use β-galactosidase having optimum pH within that range. Though the efficiency of enzyme reaction is at its best when it is performed at the optimum temperature, in order to prohibit multiplication of microorganisms in the liquid during enzyme reaction process, the reaction temperature in the present invention is preferred to be between $0^o$ - $10^oC$, and more preferably to be $5^o$ - $10^oC$.

As to dialysis membrane to be used in the present invention, a variety of dialysis membrane sold in the market may be utilized, for example, seamless cellulose tubing (by Visking), cellulose nitrate membrane or cellulose acetate membrane (both by Schleicher & Shull), cellulose acetate nitrate membrane or cellulose paper (by Celman), polyvinyl alcohol dynel nylon membrane and the like. It is necessary that the dialysis membrane is strong enough for industrial use, that it may stand the pasteurizing condition at $60^oC$ for 30 minutes or equivalent pasteurizing conditions, e.g., at $115^oC$ for 15 minutes in an autoclave or chemical sterilization by aqueous hypochloric acid solution, and that the dialyzing capability thereof will not be affected by these pasteurization or sterilization conditions. For example the cellulose tube mentioned above has pores in the order of $24\overset{o}{A}$ in diameter during its use and bears well in the sterilization treatment process at $115^oC$ for 15 minutes.

In the most simple embodiment of the present invention, these dialysis membrane or tube can be used in such a manner that those are shaped into a bag or tube at least one end of which is sealed, and it is subjected to pasteurization or sterilization, liquid containing β-galactosidase is poured thereinto, and then it is dipped into a tank in which liquid containing lactose is filled. Also these dialysis membrane or tube may be utilized in a variety of forms such as a cylindrical shape with reinforcing element made of a suitable material such as synthetic resin, stainless steel and the like and in the form of a baffle board having finely jagged surface for partitioning a dialysis tank into many compartments. As will be discussed in detail hereinafter, the surface area of the dialysis membrane through which liquid containing β-galactosidase faces to liquid containing lactose is desirable as large as possible.

The liquid containing lactose used in the present invention may be the one or more than two kinds of aqueous solutions of the materials selected from the group consisting of lactose powder, skim milk powder, whole milk powder, whey powder, demineralized whey powder, butter milk powder or one or mixture of more than two kinds of materials selected from the group consisting of whole milk, skim milk, whey, butter milk or condensed liquid thereof or one or mixture of more than two kinds of materials selected from the group consisting of deproteinized whey which is obtainable by removing protein from whey by ultrafiltration and the like, deproteinized whole milk, deproteinized skim milk, deproteinized butter milk and concentrated liquid thereof.

Solubility of lactose to water is 20 % at 30$^o$C and 13 % at 10$^o$C. Therefore, lactose concentration in the liquid containing lactose used in the present invention is proper when it is in general less than 20 % (by weight, hereinafter the same). This lactose concentration is limited depending upon the enzyme reaction temperature, reaction time and efficiency of lactose hydrolysis on the viewpoint for minimizing multiplication of microorganisms, and is also limited depending upon the concentrations of the components other than lactose such as milk protein, milk fat and the like which may be contained in the liquid containing lactose. Moreover, in the case of continuous enzyme reaction, lactose concentration is also limited on the viewpoint of ensuring continuous and homogeneous supply of the liquid containing lactose. Considering these viewpoints, preferable range of lactose concentration is 4 - 10 %. The liquic containing lactose is used after it is subjected to pasteurization or sterilization by the conventional method and then adjusted to a desired temperature.

There are a variety of embodiments for practicing the present invention, some of the typical embodiments will be described hereinafter.

The first embodiment is as follows:

A given amount of the liquid containing lactose having a given lactose concentration is prepared, and subjected to pasteurization or sterilization by a conventional method and is lead to a tank where it is kept at a temperature close to the optimum temperature for the enzyme used or alternatively at or below 10°C. A dialysis membrane is shaped into one or more of bags or elongated tubes one end of each of which is sealed and those are subjected to pasteurization or sterilization by the conventional method. The liquid prepared by dispersing and dissolving a certain amount of β-galactosidase into sterilized water is poured into said bags or tubes, and those are preliminarily and sufficiently subjected to dialysis against water to remove permeable contents contained in β-galactosidase. The amount of β-galactosidase is properly determined depending upon the lactose content in the liquid to be treated and predetermined temperature and time for the treatment. In the liquid containing lactose previously prepared, one or more of the bags or tubes which contain the liquid containing β-galactosidase are dipped, and the enzyme reaction is performed while the liquid containing lactose is agitated. In this process, the temperature and the duration for the treatment is relatively determined in order to prevent microorganisms from multiplicating.

The second embodiment is as follows:

A dialysis column having an inlet port at its lower portion and an outlet port at its upper portion and one or more of-containers of dialysis membrane each of which is shaped with a reinforcing element in a cylindrical form having a smaller diameter than the inner diameter of the column are prepared. The liquid containing β-galactosidase which is prepared in the same manner as in the first embodiment is poured into said one or more of containers of dialysis membrane and those are preliminarily and sufficiently subjected to dialysis against

- 13 -

water as mentioned above. Then said one or more of containers
filled with the liquid containing β-galactosidase are inserted
into the column, and then the liquid containing lactose which
is prepared in the same manner as in the first embodiment is
continuously supplied from the inlet port of the column and
continuously let out from the outlet port. A plurality of
such unit may be·connected in series, and the lactose-hydrolyzate
liquid may be continuously obtained.

The third embodiment is as follows:

Dialysis membrane units which are constituted with the
dialysis membrane and the reinforcing element in the form of
a buffle plate are oriented in jaxtaposition with narrow spaces
to give more than two·partitioned and water-tight chambers just
like as an ion exchange membrane electrodialytic apparatus
which are extensively employed, and the liquid containing
β-galactosidase is filled in or fed through alternate
chambers, and the liquid containing lactose is slowly fed
through the remaining alternate chambers. The liquid containing lactose passed through the above apparatus may be recirculated into the same dialysis tank, if required, or
alternatively an additonal similar dialysis tank is provided
and connected to the firstly mentioned one and the liquid
containing lactose which passed through the·first tank may be
led to the second one. Also the liquid containing β-
galactosidase may be recirculated. As for preparation and
pasteurization of the liquid containing lactose, pasteurization
of the dialysis membrane, preparation and preliminal dialysis
of the liquid containing β-galactosidase, they are the same as
in the first and second embodiments.

- 14 -

In any one of these embodiments, the liquid subjected to enzyme treatment can be yielded as lactose-hydrolyzate liquid after it is subjected to pasteurization by the conventional method and chilled.  The lactose-hydrolyzate liquid may be concentrated and dried into powder by the conventional method. The skim milk in which lactose is hydrolyzed, for example, could be served for drink just as it is, and the deproteinized whey in which lactose is hydrolyzed may be utilized as a sweetening agent for foods.

Having generally described the method of the present invention, several tests and the results to exemplify the features of the present invention will follow.  Tests 1 - 3 are for determining the various conditions in the method of the present invention, and in these tests an aqueous solution of pure lactose is used, and Test 4 shows comparison between the method of the present invention and the conventional method using skim milk as the liquid containing lactose.

TEST 1:  Quantity of β-galactosidase to be used in the present invention

Six seamless cellulose tubes (by Visking), each sealed at its one end were sterilized at 115°C for 15 minutes in an autoclave.  Six different concentration of aqueous solutions of commercially available β-galactosidase respectively containing 250 mg, 125 mg, 62.5 mg, 31.25 mg, 15.26 mg and 7.81 mg of GODO-YNL (Trademark; by Godo Shusei; enzyme activity = 10,000 units/gram; optimum pH = 6.0 - 6.5; optimum temperature = 50°C) in 10 mℓ of the solution were respectively poured into the tubes, and then another ends of these tubes were sealed so that those may have $100 cm^2$ of surface area respectively. On the other hand, 5 % aqueous solution of lactose was prepared by dissolving lactose under the Japanese pharmacopoeia in pasteurized water, and distributed into 6 tanks by 100 m$\ell$ each. Into the lactose aqueous solution in each of the tanks, previously prepared 6 dialysis membrane tubes containing different quantity of β−galactosidase were dipped in one each, and were kept at $7^{o}C$ while agitating the lactose solution with magnetic stirrers. From each of the tanks, a portion of the lactose solution which was subjected to enzyme treatment was taken out with the intervals of 12, 16, 20 and 24 hours after the dipping of the tubes respectively, and lactose hydrolyzing rates in the respective portions were determined by the following manner.

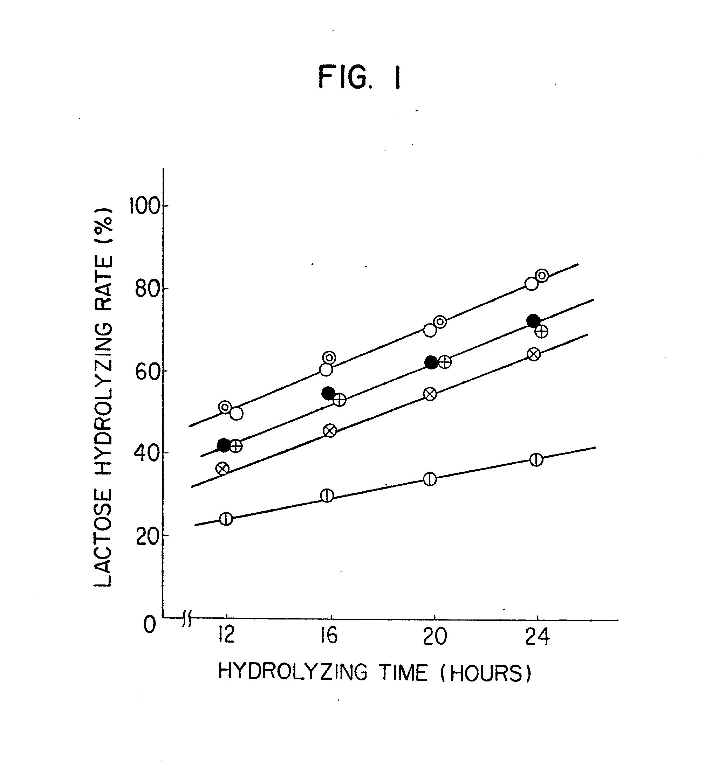

Using the commercially available reagent called "Autopack Ⓡ A·Glucose" (by Boehring Mannheim−Yamanouchi Seiyaku), quantitative determination of glucose content in the respectively taken out lactose solutions which were subjected to the enzyme treatment was carried out and then the determined value was multiplied by 2 (molecular weight of lactose is about two times as that of glucose) as the quantity of lactose hydrolyzed, and the percentages of the quantities of lactose hydrolyzed in relation to the quantity of lactose before the enzyme treatment were calculated to obtain the lactose hydrolyzing rates. In this calculation, the quantity of the substances which may migrate through the dialysis membrane was disregarded. The results were shown in Fig. 1, in which the ordinate designates the hydrolyzing rate of lactose in percent

- 16 -

and the abscissa designates the hydrolyzing time in hours.
Lines ◎ - ◎ , ○ - ○ , ● - ● , ⊕ - ⊕ , ⊗ - ⊗ and ⦶ - ⦶
respectively show the lactose hydrolyzing rate as a function of
hydrolyzing period in the cases wherein 250 mg (500 units),
125 mg (250 units), 62.5 mg (125 units), 31.25 mg (62.5 units),
15.26 mg (31.25 units) and 7.81 mg (15.63 units) of the enzyme
per gram of lactose were respectively used for the enzymatic
hydrolysis.

As will be apparent from Fig. 1, almost the same results
were obtained in the cases wherein 500 units (line ◎ - ◎ )
and 250 units (line ○ - ○ ) of the enzyme were used per gram
of lactose, and in both cases 50 % of lactose was hydrolyzed
after 12 hours and 70 % after 20 hours, and more than 80 %
after 24 hours respectively. When 125 units (line ● - ● )
and 62.5 units (line ⊕ - ⊕ ) of the enzyme were used per
gram of lactose, about 63 % and 73 % of lactose were hydrolyzed
after 20 hours and 24 hours, respectively. On the other hand,
when 15.63 units of the enzyme/gram of lactose were used
(line ⦶ - ⦶ ), the lactose hydrolyzing rate was less then 40 %
even after 24 hours. When 31.25 units of the enzyme/gram of
lactose were used (line ⊗ - ⊗ ), 55 % of lactose hydrolyzing
rate was observed after 20 hours, and 65 % after 24 hours.
In contrast to these 6 cases, a sample wherein 10 m𝑙 of an
aqueous solution containing 100 mg of the same enzyme above
was directly added to 100 m𝑙 of lactose solution prepared in
the same manner as mentioned above was subjected to the enzyme
reaction on the same condition, and lactose was completely
hydrolyzed after 8 hours.

In general, it is required that at least more than 50 % and preferably more than 60 % of lactose is hydrolyzed from the viewpoints of efficacy for the lactose-intorelent and of acceptability as a food, and it is preferable that such hydrolyzing rate is achieved within 24 hours in view of production process. Consequently, the quantity of β-galactosidase to be used in the present invention was determined as 30 - 250 units preferably 62.5 - 250 units per gram of lactose in the liquid containing lactose.

In case liquic containing lactose other than lactose solution was used, similar results were obtained as in Test 1.


TEST 2: Temperature for enzyme treatment in the present
invention

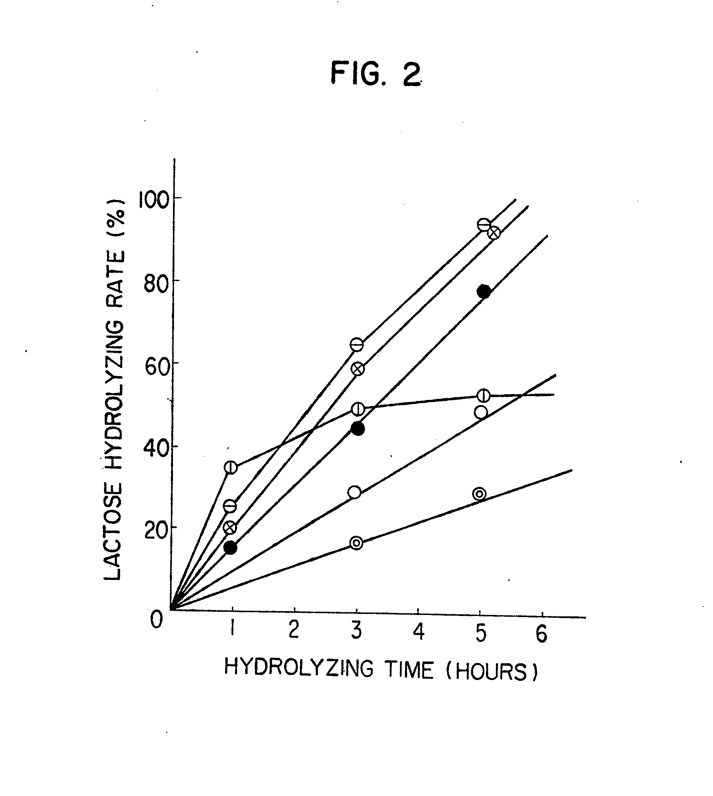

Six tanks each containing 10 $m\ell$ of 5 % aqueous lactose solution as in Test 1 were prepared. The lactose solutions in these tanks were kept at the temperatures of $10^{o}$, $20^{o}$, $30^{o}$, $40^{o}$, $50^{o}$ and $60^{o}C$ respectively. In 6 tubes made of the same dialysis membrane as used in Test 1 were sealed at their respective one end, and then 10 $m\ell$ of aqueous solution containing 250 units of the same β-galactosidase as used in Test 1 per gram of lactose was poured respectively, and other ends of these tubes were sealed so that those may have 100 $cm^{2}$ of surface area as in Test 1. These tubes were dipped into the lactose solutions in the 6 tanks one for each tank, and these lactose solutions were kept for 5 hours at the respective temperatures while agitating them with magnetic stirrers. During the enzyme treatment, portions of the lactose solutions from each of tanks were taken out after 1, 3 and 5 hours from the commencement of the treatment, and lactose hydrolyzing rates in these samples were measured in the same manner as in Test 1. The results of this Test were shown in Fig. 2, in which the ordinate designates the hydrolyzing rate of lactose in percent and the abscissa designates the hydrolyzing rate designates the hydrolyzing time in hours. Lines ◎-◎, ○-○, ●-●, ⊕-⊕, ⊗-⊗ and ⊕-⊕ show the lactose hydrolyzing rate as a function of hydrolyzing period in the cases wherein lactose solutions were respectively subjected to enzymatic hydrolysis under the temperatures of $10^{o}C$, $20^{o}C$, $30^{o}C$, $40^{o}C$, $50^{o}C$ and $60^{o}C$. As shown in Fig. 2, the hydrolyzing rates of lactose within the enzymatic reaction temperature range of $10^{o}C - 50^{o}C$ were increased in accordance with elevation of temperature up to $50^{o}C$ which is the optimum temperature of the enzyme. When the enzymatic reaction is carried out at the temperature of more than $50^{o}C$, the hydrolyzing rate of lactose is decreased within a relatively short period.

Consequently, the temperature of enzymatic reaction is preferred at the optimum temperature of the β-galactosidase used from the viewpoint of reaction efficiency, but in order to prevent microorganisms from multiplicating during the reaction, it is preferable to be $0^{o} - 10^{o}C$.

TEST 3:   Influences on hydrolyzing rates by the surface area of the dialysis membrane and the quantity of the enzyme

Using the same cellulose tube as in Test 1, 5 tubes each sealed at its one end were prepared, and into 3 of them 5 m$\ell$ of aqueous solution containing the same quantity of the same $\beta$-galactosidase as used in Test 1 (31.25 mg, 125 units/gram of lactose in the lactose aqueous solution mentioned below) was poured respectively, and were sealed at their respective other ends so that those may have different surface areas, i.e. 25 cm$^2$, 50 cm$^2$ and 75 cm$^2$ respectively. In the remaining 2 tubes, 5 m$\ell$ of aqueous solutions contained different quantities of the same $\beta$-galactosidase as used in Test 1 (62.5 mg and 125 mg, 250 units and 500 units/gram of lactose in the lactose aqueous solution which will be mentioned below) were poured respectively, and were sealed at their respective other ends so that those may have the same surface area (25 cm$^2$).

Into 5 % of aqueous lactose solution which was prepared in the same manner as in Test 1 and distributed into 5 tanks by 50 m$\ell$ each, these 5 tubes were dipped in one for each tank, and the lactose solution in these tanks were kept at 35$^o$C for 5 hours while agitating it with magnetic stirrers. During this process a portion of the lactose solution which was subjected to enzyme treatment taken out from each tank at the invervals of 1, 3 and 5 hours after commencement of the enzyme treatment, and lactose hydrolyzation rates in these samples were measured in the same manner as in Test 1. Table 1 shows the results of this Test.

TABLE 1

| Quantity of Enzyme (units/g of lactose) | Surface Area of Cellulose Tube ($cm^2$) | Lactose Hydrolyzing Rate (%) | | |
|---|---|---|---|---|
| | | aft. 1 hr. | aft. 3 hrs. | aft 5 hrs. |
| 125 | 25 | 10 | 35 | 60 |
| 125 | 50 | 20 | 53 | 87 |
| 125 | 75 | 28 | 65 | 95 |
| 250 | 25 | 10 | 35 | 65 |
| 500 | 25 | 11 | 38 | 62 |

As will be apparent from Table 1, even if the quantities of the enzyme per gram of lactose are the same, larger lactose hydrolyzing rates are obtained as the surface areas of the cellulose tubes contained the enzyme become larger, and if the surface areas of the cellulose tubes are the same, lactose hydrolyzing rates scarcely increase.

Consequently in practice of the present invention, it is advantageous to make the surface area of the dialysis membrane containing enzyme as large as possible rather than increasing the quantity of enzyme to be used.

TEST 4: Comparison of acceptability of lactose-hydrolyzed skim milk obtained by the present invention and that of conventional method

Skim milk was pasteurized and chilled in accordance with the conventional manner. Using every 5 $\ell$ of this skim milk, 4 kinds of samples of lactose-hydrolyzate liquid, i.e. lactose-hydrolyzed skim milk were prepared as follows:

1) SAMPLE 1 (a sample prepared in accordance with the present invention using refined enzyme)

Lactose-hydrolyzate liquid was prepared in the same manner as Example 1 which will be described hereinafter. This lactose-hydrolyzate liquid was heated at 85°C for 5 minutes and then cooled (This is for making treatment conditions even as in the next Sample 2).

2) SAMPLE 2(a sample prepared in accordance with the conventional method using refined enzyme)

To 5 $\ell$ of skim milk 4.6 g of the same $\beta$-galactosidase as used in Test 1 (200 units/g of lactose) was directly added, after keeping it at 10°C for 6 hours, the enzyme was inactivated by heating it at 85°C for 5 minutes, and then cooled.

3) SAMPLE 3 (a sample prepared in accordance with the present invention using crude enzyme)

Lactose-hydrolyzate liquid was prepared in similar manner as in Example 1 except that 9.2 g of crude enzyme powder (enzyme activity: 5,000 units/gram, optimum

- 22 -

0020781

pH 6.0, optimum temperature 40°C) prepared in the manner described hereinafter was used, that the crude enzyme was dissolved in 300 mℓ of distilled water and that the enzyme reaction was performed at 37°C for 5 hours. The lactose-hydrolyzate liquid was heated at 85°C for 5 minutes and then cooled likewise as in Sample 1.

Though it was apprehended in preparation of this sample that dilution of substances such as minerals in the skim milk may occur due to migration of such substances into the enzyme containing liquid, since distilled water is used for dissolving the enzyme, it was confirmed by atomic absorption spectroscopy that there was no remarkable change in minerals in the skim milk as a result of quantification of mineral composition either before or after the enzyme treatment (Table 2).

TABLE 2

|  | before enzyme treatment | after enzyme treatment |
|---|---|---|
| Na | 0.050 (%) | 0.0498 (%) |
| K | 0.150 | 0.1499 |
| Ca | 0.110 | 0.1092 |
| P | 0.090 | 0.0897 |

The crude enzyme was prepared as follows:

To the mixture of 10 g of peptone (by Kyokuto Seiyaku), 5 g of yeast extract powder (by Ebios Kogyo), 25 g of lactose, 2 g of potassium primary phosphate, 3 g of anhydrous solidum acetate, 0.2 g of magnetisum sulfate and 0.3 g of manganese sulfate (all guaranteed reagent, by Wako Junyaku), 1 $\ell$ of deionized water was added, and the pH of the thus obtained solution was adjusted to 6.8 by adding 30 % aqueous solution of sodium hydroxide, and the solution was sterilized in the conventional manner to obtain a culture medium. Using this culture medium Lactobacillus helveticus (ATCC 8018) having an ability of production of β-galactosidase was cultivated to obtain a bulk starter. To 15 $\ell$ of the sterilized culture medium having the same composition, the bulk starter was inoculated in a ratio of 3 %, and the inoculated culture medium was cultivated at 37°C for 18 hours in a jar fermentor. Centifuging the thus obtained culture at 15,000 rpm, 300 m$\ell$ of concentrated culture (solids content 10 %) was obtained. The cells contained in the concentrated culture were disrupted, and after adding thereto the same quantity of skim milk pasteurized in the conventional manner, the thus obtained mixture was freeze-dried, and 54 g of crude enzyme powder was obtained.

4) SAMPLE 4 (a sample prepared in accordance with the conventional method using crude enzyme)

This sample was prepared in similar manner as in Sample 2 except that 9.2 g of the crude enzyme previously mentioned was used and that the enzyme reaction was performed at $37^{o}C$ for 1 hour.

Combining these samples into three groups, a combination of Samples 1 and 2 (Group I), a combination of Samples 2 and 3 (Group II) and a combination of Samples 3 and 4 (Group III) were subjected to organoleptic test using three-sample test method and paired-comparison test method ("Application of Stochastics ot Chemistry and Biology, The third collection, Stochastics of the field of research", The Field of Chemistry, Extra Number, pp 144 and 145, Nankodo, 1959). In the organoleptic test, temperature of the samples were adjusted to $10^{o}C$.

(a) Organoleptic Test by Three-Sample Test

An arbitrarily arranged set of three samples which contain the same two samples (hereinafter referred to as paired samples) and odd sample (hereinafter referred to as a non-paired sample) which is different from the paired samples was prepared with respect to each of said Groups, and thus prepared three sets of three samples were tested by observers (consisting of 20 men and 20 women). The observers were required to indicate the non-paired sample in each set (Triangle Test) and to

- 25 -

answer which sample of the paired samples and the
non-paired sample they preferred (Triangle Directional
Test).  The judgement of organoleptic test between
two kinds of samples constituting each group was
made depending upon the results of triangle directional test by those observers who correctly
indicated the non-paired samples.  The results of
these tests are shown in Table 3.

TABLE 3

| Sample Group No. | Sample No. | Sex Distinction | Triangle Test | | | Triangle Directional Test | | |
|---|---|---|---|---|---|---|---|---|
| | | | Correct Answerer | Incorrect Answerer | To-tal | One who prefers smaller numbered sample | One who prefers larger numbered sample | Correct Answerer on Triangle Test |
| I | 1&2 | M | 11 | 9 | 20 | 6 | 5 | 11 |
| | | F | 10 | 10 | 20 | 5 | 5 | 10 |
| II | 2&3 | M | 10 | 10 | 20 | 6 | 4 | 10 |
| | | F | 8 | 12 | 20 | 4 | 4 | 8 |
| III | 3&4 | M | 20 | 0 | 20 | 20 | 0 | 20 |
| | | F | 20 | 0 | 20 | 20 | 0 | 20 |

- 26 -

As will be apparent from Table 3, there is no significant difference in the judgement on the samples in Groups I and II. That is, it has been found that there is no significant difference in the judgement on the lactose-hydrolyzed skim milk prepared in accordance with the present invention using refined and crude enzymes (Samples No. 1 and No. 3) and the one prepared in accordance with the conventional method using refined enzyme (Sample No. 2). In contrast, there is distinct difference in the judgement between the samples belonging to Group III. That is, there was very remarkable difference in the judgement on the lactose-hydrolyzed skim milk prepared in accordance with the present invention using crude enzyme (Sample No. 3) and the one prepared in accordance with the conventional method using crude enzyme (Sample No. 4), and many of the observers pointed out bitterness as the reason why they do not like Sample No. 4.

Judging from the results of these Tests, it is clear that remarkably palatable product can be obtained in accordance with the present invention, efen if crude enzyme which contains proteolytic enzyme and the like is used.

(b) Organoleptic Test by Paired-comparison Test

Two kinds of samples belonging to each group were tested once for each Group by observers (consisting of 25 men and 25 women). The observers were required to answer which of the two samples was preferred, and depending on their answers the judgement of the organoleptic test was made. The results of this test is shown in Table 4.

TABLE 4

| Sample Group No. | Sample No. | Sex Distinction | One who prefers smaller numbered Sample | One who prefers larger numbered Sample | Total |
|---|---|---|---|---|---|
| I | 1&2 | M | 12 | 13 | 25 |
| | | F | 14 | 11 | 25 |
| II | 2&3 | M | 13 | 12 | 25 |
| | | F | 12 | 13 | 25 |
| III | 3&4 | M | 25 | 0 | 25 |
| | | F | 25 | 0 | 25 |

As is clear from the Table 4, similar results were obtained as in the organoleptic test in (a) above.

EXAMPLE 1

To 300 m$\ell$ of pasteurized skim milk (pH 6.7, lactose content 4.6 %), 4.6 g of commercially available β-galactosidase, GODO-YNL (Trademark; by Godo Shusei; Enzyme Activity 10,000 units/gram, optimum pH 6 - 6.5; optimum temperature 50$^{\circ}$C) was added in aseptic atmosphere. Thus obtained liquid was distributed into 10 cellulose tubes (by Visking) each sealed at its one end by 30 m$\ell$ each in aseptic atmosphere and then the respective other ends were sealed (total surface area 4,000 cm$^2$). Into 5 $\ell$ of the same pasteurized skim milk in a tank with a stirrer as mentioned above, these 10 cellulose tubes were dipped (200 units/gram lactose), and after keeping the skim milk at 10$^{\circ}$C for 20 hours under agitation, they were removed. Thus remarkably palatable skim milk, in which 80 % of lactose was hydrolyzed, was obtained.

EXAMPLE 2

Commercially available whey powder was dissolved into water at the concentration of 6.5 % to obtain 5 $\ell$ of reconstituted whey (pH 6.4, lactose content 4.6 %), and thus obtained reconstituted whey was poured into a tank with a stirrer and was heated at 70$^{\circ}$C for 5 minutes for pasteurization, and then cooled to 10$^{\circ}$C. Into this solution, the 10 cellulose tubes containing β-galactosidase (200 units/gram of lactose) which were used in Example 1 were dipped for repeated use. Then the whey was kept at 10$^{\circ}$C for 20 hours during agitation. Thus remarkably palatable lactose-hydrolyzed whey, in which 80 % of the lactose was hydrolyzed, was obtained.

EXAMPLE 3                                    0020781

Into a tank with a stirrer, 5 $\ell$ of the mixture consisting of 2.5 $\ell$ of reconstituted skim milk prepared by dissolving commercially available skim milk powder into water at a concentration of 10 % and 2.5 $\ell$ of raw milk (pH 6.6, lactose content 4.5 %) was poured, and after pasteurization by heating at 80°C for 10 minutes, the mixture was cooled to 5°C.

On the other hand, the solution with 3.0 g of commercially available β-galactosidase (133 units/gram of lactose) which was used in Example 1 was added aseptically into 500 m$\ell$ of pasteurized distilled water. This solution was distributed 50 m$\ell$ each, in aseptic atmosphere, to 10 sterilized cellulose tubes sealed at their respective one ends as were in Example 1, and then sealed at their respective other ends (total surface area 4,000 cm$^2$). These 10 tubes were dipped into the tank in which 5 $\ell$ of the mixture consisting reconstituted skim milk and raw milk, and the mixture was kept at 5°C for 20 hours under agitation. Thus remarkably palatable lactose-hydrolyzed milk, in which about 70 % of lactose was hydrolyzed, was obtained.


EXAMPLE 4

To 500 m$\ell$ of pasteurized distilled water, 4.5 g of commercially available β-galactosidase called Maxi lact "20,000" (Trademark, by Gist-Brocades, enzyme activity 10,000 units/gram, optimum pH 6.5 - 7.0, optimum temperature 35° - 40°C) was added and dissolved in an aseptic atmosphere. This enzyme solution was distributed 50 m$\ell$ each to 10

pasteurized cellulose tubes sealed at their respective one ends as were used in Example 1, and then sealed at their respective other ends (so as to the total surface area be 4,000 $cm^2$).

On the other hand, 5 $\ell$ of two-fold condensed skim milk (pH 6.6, lactose content 9.0 %) was poured into a tank with a stirrer, and after pasteurizing it at $80^oC$ for 10 minutes, it was cooled to $35^oC$. Into this condensed skim milk, 10 cellulose tubes containing the enzyme solution were dipped (100 units/gram of lactose) and kept at $35^oC$ for 5 hours under agitation. Thus remarkably palatable lactose-hydrolyzed condensed milk, in which about 60 % of lactose was hydrolyzed, was obtained.

EXAMPLE 5

To 600 m$\ell$ of tap water, 50 g of unrefined β-galactosidase powder (enzyme activity 5,000 units/gram, optimum pH 6.0, optimum temperature $40^oC$) as in Example 4 was added and dissolved. This enzyme solution was distributed 30 m$\ell$ each to 20 pasteurized cellulose tubes (by Visking) which were sealed at their respective one ends, and then sealed their respective other ends (so as to the total surface area be 8,000 $cm^2$).

On the other hand, cheese whey solution from which protein was removed by ultra-filtration which pH was adjusted to 6.4 using 30 % aqueous solution of sodium hydroxide was condensed into the two-fold by a vacuum concentrator. Into a tank with a stirrer, 10 $\ell$ of this two-fold condensed

- 31 -

protein-free cheese whey (pH 6.4, lactose content 10 %) was poured, and after pasteurizing at 70°C for 5 minutes, the whey was cooled to 37°C.  In this tank, the 20 cellulose tubes containing enzyme solution were dipped (250 units/gram of lactose) the whey was kept at 37°C for 3 hours under agitation. Thus remarkably palatable lactose-hydrolyzate liquid, in which about 50 % of lactose was hydrolyzed, was obtained.

1.     A method for preparation of liquid containing lactose-hydrolyzate, which is characterized in that:

partitioning a vessel into more than two compartments by at least one dialysis membrane;

alotting these compartments to liquid containing lactose and liquid containing β-galactosidase so that these two different liquids being adjacent to each other; and

thereby hydrolyzing lactose in the liquid containing lactose and obtaining the liquid containing lactose-hydrolyzate without inclusion of β-galactosidase.

2.     A method of claim 1, which is characterized in that:

said one or more of compartments allotted to the liquid containing β-galactosidase comprises one or more of sealable containers made of dialysis membranes, and said one or more of containers are sealed after being filled therein with the liquid containing β-galactosidase and then dipped in the liquid containing lactose.

3.     A method of claim 1, which is characterized in that:

the liquid containing lactose is slowly and continuously fed into the vessel from its one end; and

said one or more of chambers allotted to β-galactosidase are so arranged as those do not prohibit the gradual feeding of the liquid containing lactose into the vessel.

4.    A method of claim 3, which is characterized in that:

at least one additional vessel is further provided;

the liquid containing lactose is continuously and slowly fed into the vessel from its one end to its other end;

said one or more of chambers allotted to the liquid containing β-galactosidase are arranged in such a manner that those do not prohibit the gradual feeding of the liquid containing lactose into the vessel; and

said vessel being connected in series to said at least one additional vessel.

5.    A method according to any one of claims 1 - 4, characterized in that:

said liquid containing lactose is one or mixture of more than two, selected from the group consisting of whey, demineralized whey, skim milk, whole milk, butter milk, deproteinized whey, deproteinized skim milk, deproteinized butter milk, condensed liquid thereof, reconstituted demineralized whey, reconstituted skim milk and reconstituted whole milk.

6.    A method of claim 5, which is characterized in that:

the liquid containing lactose includes 20 - 4 % of lactose content.

7.    A method of claim 5, which is characterized in that:

the quantity of β-galactosidase used in said one vessel is 30 - 250 units/gram of lactose in the total amount of the liquid containing lactose in said one or more of the

compartments allotted to the liquid containing lactose in the vessel.

8.    A method of claim 7, which is characterized in that:
the dialysis membrane interposing between the liquid containing lactose and the liquid containing β-galactosidase is arranged in such a manner that the surface area of the dialysis membrane facing the liquid containing lactose be substantially maximum with respect to the volume of the liquid containing β-galactosidase used.

9.    A method of claim 5, which is characterized in that:
the enzyme reaction is performed within the temperature range of $0^o - 10^oC$.

SCOPE OF THE CLAIMS

1.　(amended)

A method for preparation of edible liquid containing lactose-hydrolyzate, which is characterized in that:

partitioning a vessel into more than two compartments by at least one dialysis membrane;

alotting these compartments to edible liquid which contains lactose to a cencentration of 20 - 4 % by weight, water and other substances, and liquid which contains 30 - 250 units of β-galactosidase per gram of lactose in said edible liquid so that these two different liquids are adjacent to each other; and

selectively hydrolyzing lactose by allowing the lactose in said edible liquid to permeate into the β-galactosidase containing liquid and then by allowing the resulted lactose hydrolyzate to migrate back to the initial lactose containing liquid through the interposing dialysis membrane, whereby liquid containing lactose-hydrolyzate is obtained without inclusion of β-galactosidase, without substantially adding any substances thereto and without substantially removing any substances therefrom except that lactose is hydrolyzed.

2.　A method of claim 1, which is characterized in that:

said one or more of compartments allotted to the liquid containing β-galactosidase comprises one or more of sealable containers made of dialysis membranes, and said one or

- 36 -　　　　　(for substitution)

0020781

more of containers are sealed after being filled therein with the liquid containing β-galactosidase and then dipped in the liquid containing lactose.

3. A method of claim 1, which is characterized in that:
the liquid containing lactose is slowly and continuously fed into the vessel from its one end; and
said one or more of chambers allotted to β-galactosidase are so arranged as those do not prohibit the gradual feeding of the liquid containing lactose into the vessel.

4. A method of claim 3, which is characterized in that:
at least one additional vessel is further provided;
the liquid containing lactose is continuously and slowly fed into the vessel from its one end to its other end;
said one or more of chambers allotted to the liquid containing β-galactosidase are arranged in such a manner that those do not prohibit the gradual feeding of the liquid containing lactose into the vessel; and
said vessel being connected in series to said at least one additional vessel.

5. A method according to any one of claims 1 - 4, characterized in that:
said liquid containing lactose is one or mixture of more than two, selected from the group consisting of whey, demineralized whey, skim milk, whole milk, butter milk, deproteinized whey, deproteinized skim milk, deproteinized

- 37 -                    (for substitution)

butter milk, condensed liquid thereof, reconstituted demineralized whey, reconstituted skim milk and reconstituted whole milk.

6.    (deleted)

7.    (deleted)

8.    (amended)

A method of claim 5, which is characterized in that:

the dialysis membrane interposing between the liquid containing lactose and the liquid containing β-galactosidase is arranged in such a manner that the surface area of the dialysis membrane facing the liquid containing lactose be substantially maximum with respect to the volume of the liquid containing β-galactosidase used.

9.    A method of claim 5, which is characterized in that:

the enzyme reaction is performed within the temperature range of $0^\circ - 10^\circ C$.

(for substitution)

EXPLANATION BASED ON SECTION 19 (1) OF PCT

1.    In order to distinguish the present invention from
4 references cited in the International Search Report mailed
on February 4, 1980, Claim 1 in the present application was
amended so that it comes to be more restrictive and clearer,
and Claims 5 and 6 were cancelled.  By this amendment, the
specification of the present application should be read as to
disclose the invention having following features.

(1)     A method for preparation of liquid containing
lactose-hydrolyzate which is served for diet in the form
of drink or others is provided wherein lactose contained
in the liquids containing lactose having complicated
system such as whole milk, skim milk, whey and the like is
selectively hydrolyzed without using troublesome means
such as immobilization of enzyme, pressurization of the
liquid to be treated and the like and moreover without
changing the inherent nature and acceptability of the
liquid containing lactose to be treated.

(2)     By the amendment mentioned above, aqueous solution of
pure lactose is no longer included in the liquid containing lactose to be treated, and the liquid containing
lactose used in the present invention contains 20 - 4 %
(by weight) of lactose and water and other substances
such as protein, fat, minerals and other tasting
substances.

(3)    Lactose contained in the liquid containing lactose
to be treated may permeate into the liquid containing
β-galactosidase through the dialysis membrane, and after
hydrolyzation of lactose, hydrolyzate of lactose may
migrate back to the initial liquid containing lactose
through interposing dialysis membrane without using any
specific means such as pressurization of the liquid to be
treated and the like.

(4)    The liquid containing lactose to be treated is the
one which may be served as a diet in the form of liquid or
others, and by hydrolysis of lactose the treated liquid is
not subjected to any affection except that lactose is
hydrolyzed.

Consequently appearence, physical nature, flavor and
taste of the liquid of the raw material is not changed
and the inherent nature and taste of the liquid containing
lactose is substantially maintained.

(5)    Lactose contained in the liquid containing lactose
is hydrolyzed by β-galactosidase in the present invention,
even if crude β-galactosidase is used, lactose is hydrolyzed into glucose and galactose and those are never
decomposed further into the substances having lesser
molecular weights, and the components other than lactose
such as protein and other tasting substances are not
affected by the enzyme treatment.

(6)    β-galactosidase is not  immobilized but used in
a solution, it is separated from the liquid to be treated by dialysis membrane, and therefore β-galactosidase
does not migrate to the treated liquid.

(7)     Relatively small amount of β-galactosidase (30 - 250
units) with respect to lactose content in the liquid to
be treated may be used.

II.     The claims as amended avoid the connection of the
present invention to the cited references as follows:

(1)   regarding the first cited reference:
The connection is avoided on the points mentioned
in the above I (1), (2), (4) and (5).

(2)   regarding the second cited reference:
The connection is avoided on the points mentioned
in the above I (1), (4) and (5).

(3)   regarding the third cited reference:
The connection is avoided on the points mentioned
in the above I (1), (3), (4) and (5).

(4)   regarding the fourth cited reference:
The connection is avoided on the points mentioned
in the above I (1), (3), (4), (5), (6) and (7).

FIG. 1

# FIG. 2

# INTERNATIONAL SEARCH REPORT

International Application No PCT/JP79/00298

## I. CLASSIFICATION OF SUBJECT MATTER (if several classification symbols apply, indicate all) 3

According to International Patent Classification (IPC) or to both National Classification and IPC

A23C 21/02, A23C 9/12, A23C 17/02, C12P 1/00
A23L 1/236

## II. FIELDS SEARCHED

### Minimum Documentation Searched 4

| Classification System | Classification Symbols |
|---|---|
| I P C | A23C 21/02, A23C 9/12, A23C 17/02, C12Q 1/00, C12M 1/40 |

Documentation Searched other than Minimum Documentation
to the Extent that such Documents are Included in the Fields Searched 5

## III. DOCUMENTS CONSIDERED TO BE RELEVANT 14

| Category * | Citation of Document, 16 with indication, where appropriate, of the relevant passages 17 | Relevant to Claim No. 18 |
|---|---|---|
| X | JP, A, 49-95690, 1974-9-11, See column 1, lines 5 to 10, page 519, Fig. 2   Matsumoto Tetsuro | 1-9 |
| X | JP, A, 48-68772, 1973-9-19, See left column, lines 5 to 11 Morinaga Nyugyo Kabushiki Kaisha | 1-9 |
| X | JP, A, 52-1089, 1977-1-6, See left column, page 536, lower right column, lines 2 to 6 Kanegafuchi Kagaku Kogyo Kabushiki Kaisha | 1-9 |
| X | JP, A, 47-16663, 1972-9-2, See page 416, column 13, lines 4 to 7, page 417, column 17, line 14 to column 18, line 9, page 414, column 5, lines 6 to 8 Washington Biochemical Corp. | 5, 7, 9 |

* Special categories of cited documents: 15

"A" document defining the general state of the art

"E" earlier document but published on or after the international filing date

"L" document cited for special reason other than those referred to in the other categories

"O" document referring to an oral disclosure, use, exhibition or other means

"P" document published prior to the international filing date but on or after the priority date claimed

"T" later document published on or after the international filing date or priority date and not in conflict with the application, but cited to understand the principle or theory underlying the invention

"X" document of particular relevance

## IV. CERTIFICATION

| Date of the Actual Completion of the International Search 2 | Date of Mailing of this International Search Report 2 |
|---|---|
| January 23, 1980 (23.01.80) | February 4, 1980 (04.02.80) |
| International Searching Authority 1 | Signature of Authorized Officer 20 |
| Japanese Patent Office | |

Form PCT/ISA/210 (second sheet) (October 1977)